# FASCICULE DE BREVET EUROPEEN

(11) **EP 2 068 810 B1**
(45) Date de publication et mention de la délivrance du brevet: **11.08.2010**
(21) Numéro de dépôt: 07858408.3
(22) Date de dépôt: 08.10.2007
(51) Int. Cl.: A61H 23/04, A61N 1/32

(54) **PROCÉDÉ ET APPAREIL POUR LA RÉDUCTION DES SURCHARGES ADIPEUSES**
VERFAHREN UND VORRICHTUNG ZUR REDUZIERUNG ÜBERSCHÜSSIGEN ADIPÖSEN GEWEBES
METHOD AND APPARATUS FOR REDUCING EXCESS ADIPOSE TISSUE

(30) Priorité: 06.10.2006 FR 0608815
(43) Date de publication de la demande: 17.06.2009
(73) Titulaire: H.C.A SA, 1219 Luxembourg (LU)
(72) Inventeur: GREFF, Daniel, 78490 Mere (FR)
(74) Mandataire: Laget, Jean-Loup
(86) Numéro de dépôt international: PCT/FR2007/001639
(87) Numéro de publication internationale: WO 2008/043907

(56) Documents cités:
- WO-A-2006/103707
- WO-A1-02/087692
- FR-A- 2 616 064
- FR-A1- 2 684 547
- FR-A1- 2 855 415

## Description

L'invention concerne un dispositif pour la réduction des surcharges adipeuses comme défini dans la revendication 1.

Le document FR 2 855 415 décrit un dispositif électrique pour la réduction des surcharges adipeuses comportant des sangles isolantes dans lesquelles un conducteur est disposé en brins successifs sensiblement parallèles entre eux, le conducteur étant parcouru par un courant alternatif de tension basse et de fréquence basse. Le champ électromagnétique créé par le courant alternatif a une action lipolytique et calorigène.

Cependant, malgré des résultats certains sur la réduction de la masse grasse corporelle, l'efficacité de ce dispositif reste limitée.

L'un des buts de l'invention est de proposer un appareil pour la réduction des surcharges adipeuses qui associe à l'action du champ électromagnétique une action complémentaire purement physique, non traumatisante, non invasive, mais efficace.

Dans la suite de la description, il est également divulgué un procédé de réduction des surcharges adipeuses, caractérisé en ce que les zones adipeuses du corps sont soumises simultanément à une lipolyse, par l'action d'un appareil générateur d'ondes électromagnétiques à basse fréquence, et à une extraction, hors des cellules adipeuses, des acides gras libérés par la lipolyse, et à leur drainage vers le système vasculaire et lymphatique, par l'action d'un appareil de pressothérapie.

Avantageusement, les ondes électromagnétiques ont une fréquence inférieure à 100 Hz, et l'appareil de pressothérapie est à double gradient de pression.

La description divulgue également un appareil pour la mise en oeuvre du procédé précité, comportant
- un générateur d'ondes électromagnétiques à basse fréquence ;
- des sangles isolantes pour l'enveloppement des zones adipeuses du corps, équipées d'un conducteur replié en brins successifs disposés alternativement dans un sens puis dans l'autre, et
- un appareil de pressothérapie comportant deux bottes et une ceinture abdominale, et superposé auxdites sangles.

L'invention définie par la revendication 1, est décrite ci-après dans un mode de réalisation non limitatif.

L'appareil pour la réduction des surcharges adipeuses se compose essentiellement de l'association d'un sous-ensemble électrique et d'un sous-ensemble mécanique.

Le sous-ensemble électrique, analogue à celui du document FR 2 855 415 auquel il est fait référence, comprend un générateur d'ondes électromagnétiques à fréquence basse, inférieure à 100 Hz, par exemple de 50 Hz et de sangles isolantes destinées à envelopper les zones adipeuses du corps au niveau des bras, de l'abdomen, des cuisses et des mollets. Ces sangles sont équipées chacune d'un conducteur replié en brins successifs disposés alternativement dans un sens puis dans l'autre. Le conducteur est incorporé dans un tissu isolant thermostable, recouvert d'une protection plastique ignifuge.

Les conducteurs sont alimentés par le générateur, sous basse tension, 40 V par exemple, et sous basse fréquence, 50 Hz par exemple.

Le passage du courant dans les conducteurs crée un champ électrostatique à la surface des sangles et un champ électromagnétique, qui stimulent les mécanismes biologiques de la lipolyse, assurant ainsi une réduction sélective de la masse grasse corporelle.

Le sous-ensemble mécanique est un appareil de pressothérapie à double gradient de pression. Il se compose de deux bottes et d'une ceinture abdominale. Chacune des bottes comprend par exemple 10 cellules, et la ceinture, comprenant par exemple 3 cellules, est reliée aux bottes. Le sous-ensemble de pressothérapie est superposé aux sangles du sous-ensemble électrique.

Le double gradient de pression correspond d'une part à la diminution progressive de la pression entre la première cellule, au niveau du pied, et la dernière cellule, en haut de la botte, d'autre part à l'augmentation progressive de la pression dans chaque cellule au fur et à mesure des cycles de gonflage.

Cette technique du gradient de pression évite l'effet de garrot produit par les appareils de pressothérapie sans gradient de pression.

Le fonctionnement de l'appareil pour la réduction des surcharges adipeuses s'analyse de la manière suivante :
- le sous-ensemble électrique stimule la lipolyse, c'est-à-dire le déstockage des graisses par libération des acides gras, au sein des cellules adipeuses ;
- le sous-ensemble mécanique, par effet de pression mécanique, améliore l'évacuation des acides gras hors des cellules adipeuses et draine les acides gras libérés dans le tissu adipeux vers le système vasculaire et lymphatique.

La lipolyse est un processus biologique complexe. Elle est sous la dépendance de deux types de capteurs membranaires adipocytaires :
- les récepteurs alpha-adrénergiques qui commandent le stockage des graisses dans les adipocytes ;
- les récepteurs béta-adrénergiques qui commandent le déstockage des graisses contenues dans les adipocytes.

Les ondes électromagnétiques à basse fréquence (50 Hz) sont capables de stimuler spécifiquement les récepteurs béta-adrénergiques, et favorisent ainsi, de manière biologique, la lipolyse adipocytaire. Ces ondes agissent de façon non invasive, inoffensive et indolore, pour assurer une fonction amincissante.

A cette fonction amincissante du sous-ensemble électrique est associée la fonction drainante du sous-ensemble mécanique, les deux sous-ensembles fonctionnant simultanément.

La technique de pressothérapie à double gradient de pression permet en effet une bonne extraction, hors de la cellule adipeuse, des acides gras libérés par la lipolyse, et une bonne évacuation de ces acides gras vers le système vasculaire et lymphatique. Cette technique, qui opère un massage par compression et décompression, active la circulation sanguine et lymphatique et contribue au drainage des acides gras qui ont été libérés par la lipolyse.

A titre indicatif, des tests réalisés sur un groupe de 36 sujets, à raison de 2 séances hebdomadaires d'une heure pendant 6 semaines, en l'absence de massage et de régime alimentaire, ont permis de constater une perte moyenne en poids de 6,2% et une perte moyenne en masse grasse corporelle de 12,4%, ce qui traduit une diminution très nette des surcharges adipeuses.

Ces tests montrent une réduction quasi spécifique de la masse grasse corporelle sans modification significative de la masse maigre. Il s'agit donc bien d'un processus d'amincissement, qui ne concerne que les surcharges adipeuses, et non d'un processus d'amaigrissement.

## Revendications

1. Appareil pour la réduction des surcharges adipeuses comportant :
- des sangles isolantes pour l'enveloppement des zones adipeuses du corps au niveau des bras, de l'abdomen, des cuisses et des mollets, lesdites sangles étant équipées d'un conducteur replié en brins successifs disposés alternativement dans un sens puis dans l'autre, ledit conducteur étant relié à un générateur d'ondes électromagnétiques ayant une fréquence inférieure à 100Hz ;
- un appareil de pressothérapie comportant deux bottes et une ceinture abdominale reliée aux bottes, lesdites bottes et ceinture étant superposées aux sangles et comprenant des cellules de gonflage ;
**caractérisé en ce que** l'appareil de pressothérapie est à double gradient de pression, le double gradient de pression correspondant d'une part à la diminution progressive de la pression entre une première cellule, au niveau du pied et une dernière cellule, en haut de la botte, d'autre part à l'augmentation progressive de la pression dans chaque cellule au fur et à mesure des cycles de gonflage.

## Claims

1. A device for the reduction of adipose overloads, comprising:
- insulating straps for the envelopment of the adipose zones of the body at the arms, the abdomen, thighs and calves, said traps being provided with a conductor folded into successive strands arranged alternately in one direction and then in the other, said conductor being connected to an electromagnetic wave generator having a frequency that is less than 100 Hz;
- a presso-therapy device comprising two boots and an abdominal belt connected to the boots, said boots and said belt being superimposed on said straps and comprising inflation cells;
**characterised in that** the presso-therapy device has a double pressure gradient, the double pressure gradient corresponding, on the one hand, to the gradual reduction of pressure between a first cell, at the level of the foot, and a last cell, at the top of the boot, and, on the other hand, to the gradual increase of the pressure in each cell as the inflation cycles proceed.

## Patentansprüche

1. Gerät für die Reduzierung von übermäßigen Fettansätzen, einschließend:
- isolierende Bände für Umwickelung der Fettzonen des Körpers an den Armen, am Bauch, an den Oberschenkeln und an den Waden, wobei die Bände mit einem von aufeinanderfolgenden Adern gefalteten Leiter ausgerüstet sind, wobei die Adern umwechselnd in eine Richtung und dann in die andere angeordnet sind, wobei der Leiter mit einem Generator von elektromagnetischen Wellen mit einem Frequenz niedriger als 100 Hz verbunden ist;
- ein Gerät für Presstherapie mit zwei Stiefeln und einem mit den Stiefeln verbunden Bauchgürtel, wobei die Stiefeln und der Gürtel die Bände überlagern und Aufblaszellen umfassen;
**dadurch gekennzeichnet, daß** das Gerät für Presstherapie ein Gerät mit doppelte Druckgradient ist, wobei die doppelte Druckgradient einerseits einer zunehmenden Druckreduzierung zwischen einer ersten Zelle, auf Ebene des Fußes, und einer letzten Zelle, oben auf dem Stiefel, und andererseits einer zunehmenden Drucksteigerung in jeder Zelle nach Maßgabe der Aufblaskreisprozessen entspricht.
